(19) Europäisches Patentamt
European Patent Office
Office européen des brevets

(11) Publication number: 0 218 816 B1

# (12) EUROPEAN PATENT SPECIFICATION

(45) Date of publication of the patent specification: 26.09.90

(51) Int. Cl.[5]: C07D 285/04, C07D 417/12, C10M 135/36

(21) Application number: 86110155.8

(22) Date of filing: 23.07.86

(54) **Thiadiazole compounds and lubricant additives thereof.**

(30) Priority: 15.10.85 US 787577

(43) Date of publication of application: 22.04.87 Bulletin 87/17

(45) Publication of the grant of the patent: 26.09.90 Bulletin 90/39

(84) Designated Contracting States: DE FR GB IT

(56) References cited:
EP-A- 0 104 667
EP-A- 0 146 087
US-A- 2 836 564
US-A- 4 193 882
US-A- 4 536 189

**The file contains technical information submitted after the application was filed and not included in this specification**

(73) Proprietor: PENNWALT CORPORATION, Pennwalt Building Three Parkway, Philadelphia Pennsylvania 19102(US)

(72) Inventor: King, James P., 904 Breezewood Lane, Lansdale Pennsylvania 19446(US)
Inventor: Hill, Billy L., Douglas Drive at Glendale, Pine Forge Pennsylvania 19548(US)

(74) Representative: Kraus, Walter, Dr. et al, Patentanwälte Kraus, Weisert & Partner Thomas-Wimmer-Ring 15, D-8000 München 22(DE)

EP 0 218 816 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel thiadiazole compounds and lubricant compositions thereof prepared from the derivatives of dimercaptothiadiazole and maleic anhydride or a halosuccinate. Lubricating compositions containing the compound, or their metal salts, provide antiwear and antioxidant properties.

Zinc dialkyldithiophosphates have been used in lubricants as antioxidant and antiwear additives for many years (CRC Handbook of Lubrication, CRC Press, Inc., 1984). However, there has been some concern regarding the presence of phosphorous-containing compounds in crankcase lubricants that may be harmful to automobile catalytic converters. It has been considered desirable to use non-phosphorous-containing additives that can provide comparable performance. The use of the derivatives of dimercaptothiadiazole, generally, in lubricants as inhibitors for corrosion and oxidation has also been long recognized (CRC Handbook of Lubrication, CRC Press, Inc., 1984). For instance, U.S. Patent No. 4 193 882 also teaches that the reaction products of 1,3,4-thiadiazole and oleic acid inhibit metal corrosion.

EP-A 0 146 087 describes certain thiazolidine derivatives for use in the processing of color photographic light-sensitive material. US-A 2 836 564 describes certain 1,3,4-thiadiazole-2,5-bis-(thia-alpha-aliphatic acids) and their use in lubricant compositions.

According to the present invention, it has been found that specific novel organosulfur compounds and their metal salts used as lubricant additives can inhibit oxidation and improve extreme pressure and antiwear characteristics of a lubricant. Zinc and molybdenum are preferred metal ions although other metals such as nickel, cobalt, iron, tin and antimony are useful.

Accordingly, present invention provides a compound of the structure

$$\left[ Z - S - Q - S - \right]_x M$$

wherein:
Q is a bivalent thiadiazole ring moiety selected from the group consiting essentially of 1,3,4-thiadiazole; 1,2,4-thiadiazole; 1,2,3-thiadiazole; and 1,2,5-thiadiazole;
Z is a succinate group of the structure

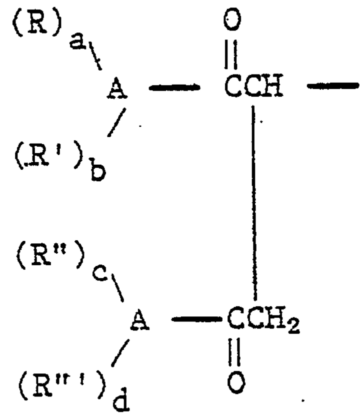

or

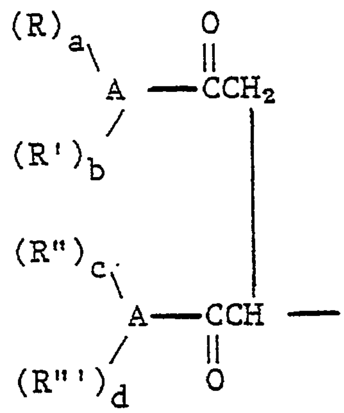

,

wherein:
A is an oxygen or nitrogen atom, with the proviso: when A is oxygen a is 1, b is zero, c is 1, and d is zero; and when A is nitrogen a, b, c, and d are each 1;
R, R′, R′, and R‴ are each independently selected from the group consisting essentially of hydrogen, alkyl of 1 through 22 carbon atoms, and branched or straight chain alkenyl of 2 through 22 carbon atoms; with the further proviso that the number one and four carbon atoms of the succinate group Z can be linked by a single A in which case when A is oxygen a, b, c, and d are zero, and when A is nitrogen a is one and b, c, and d are zero;
M is a metal ion selected from from Groups Ib to Vb, and transition elements of the Periodic Table and $MoO_2^{+2}$; and x is a whole number equal to the valence of M, as well as lubricant compositions comprising the above-described compound.

Preferred compounds are those as above defined wherein M is selected from the group consisting of zinc, copper with a valence state of 2, cobalt with a valence state of 2, $MoO_2$ with a valence state of 2, aluminum, and antimony with a valence state of 3. It is more preferred that R, R′, and R‴ are each independently selected from the group consisting essentially of decyl, tridecyl, oleyl, 2-ethylhexyl, and isocetyl.

It is most preferred that Q is the 1,3,4-thiadiazole moiety.

Preferred specific compounds of the invention are:
bis[ditridecyl 2-(2-thio-1,3,4-thiadiazol-5-ylthio)-succinate]-S-zinc(2+); bis[ditridecyl 2-(2-thio-1,3,4-thiadiazol-5-ylthio)-succinate]-S-dibutyl tin; bis[dioleyl 2-(2-thio-1,3,4-thiadiazol-5-ylthio)-succinate]-S-molybdenum dioxide; bis[ditridecyl 2-(2-thio-1,3,4-thiadiazol-5-ylthio)-succinate]-S-molybdenum dioxide; bis[ditridecyl 2-(2-thio-1,3,4-thiadiazol-5-ylthio)-succinate]-S-cobalt(2+); bis[ditridecyl 2-(2-thio-1,3,4-thiadiazol-5-ylthio)-succinate]-S-nickel(2+); bis[N,N′-dioleyl-2-(2-thio-1,3,4-thiadiazol-5-ylthio)-succinamide]-S-zinc(2+); bis[N-(2-ethylhexyl)-2-(2-thio-1,3,4-thiadiazol-5-ylthio)-succinimide]-S-molybdenum dioxide; bis[diisocetyl 2-(2-thio-1,3,4-thiadiazol-5-ylthio)-succinate]-S-zinc(2+).

The lubricant composition of the invention comprises a major amount of a grease or oil of lubricating viscosity and a minor amount of a compound as defined above as a lubricant additive to provide enhanced properties to said grease or oil.

The novel organosulfur compounds of this invention can be prepared by first reacting a 1:1 adduct of dimercaptothiadiazole and maleic anhydride with an amine or alcohol to either partially or totally convert it into an imide, amide or ester. The resulting product is then reacted with a metal salt, such as zinc oxide,

zinc carbonate, zinc acetate or zinc halide, in an organic solvent at a temperature between about 50°C and 180°C.

The extreme pressure and antiwear properties of these compounds were evaluated by means of known methods: ASTM D 2596 and ASTM D 2266, respectively. The antioxidant properties of a lubricant were obtained by a versatile thermal-oxidative method known as pressure differential scanning calorimetry (PDSC): The method measures oxidation induction time. A sample is held at an isothermal temperature in an oxidizing atmosphere. Under a set of identical conditions, the larger the oxidation induction time of a lubricant, the better is its antioxidant properties (Zeman, Stuwe and Koch Thermochimica Acta, 80, 1–9, 1984, Elsevier Science Publisher B.V.).

Example 1

Preparation of 2-(2-mercapto-1,3,4-thiadiazol-5-ylthio)-succinic anhydride:

A mixture of 95 grams (0.5 moles) of 2,5-dimercapto-1,3,4-thiadiazole and 49.0 grams (0.5 moles) of maleic anhydride in 500 ml. tetrahydrofuran was refluxed for six hours. After distilling off the solvent, a light tan solid (123 g.) was obtained (m.p. 194–196°C). Its infrared spectrum and elemental analysis appeared to be consistent with the proposed structure.
Calculated for $C_6H_4N_2O_3S_3$: C, 29.0; H, 1.61; N, 11.3; S, 38.7
Found: C, 29.0; H, 1.64; N, 11.0; S, 38.5

Example 2

Preparation of ditridecyl 2-(2-mercapto-1,3,4-thiadiazol-5-ylthio)-succinate:

A mixture of 99.2 grams (0.4 moles) of 2-(2-mercapto-1,3,4-thiadiazol-5-ylthio)-succinic anhydride, 160 grams (0.8 moles) of tridecyl alcohol and 0.2 grams of p-toluene sulfonic acid in 800 ml. of toluene was refluxed for 18 hours. This reaction was monitored by means of the amount of water collected in a Dean-Stark trap attached to the refluxing condenser. After distilling off the solvent, attempt was made to distill off the reaction product without success. The reaction product was a viscous oil. Its IR spectrum and elemental analysis was consistent with the proposed structure.
Calculated for $C_{33}H_{57}N_2O_4S_3$: C, 61.0; H, 9.05; N, 4.46; S, 15.3
Found: C, 60.7; H, 9.45; N, 4.40; S, 14.8

Example 3

Preparation of bis[ditridecyl 2-(2-thio-1,3,4-thiadiazol-5-ylthio-succinate]-S-zinc(2+):

A mixture of 6.28 grams (0.05 moles) of zinc carbonate and 31.5 grams (0.048 moles) of ditridecyl 2-(2-mercapto-1,3,4-thiadiazol-5-ylthio)-succinate in 200 ml. of toluene was refluxed for 13 hours. The reaction product was filtered to remove excess of zinc carbonate and the filtrate was subjected to distillation to remove the solvent under reduced pressures. The reaction product is an amber, viscous oil. Its IR spectrum and elemental analysis were consistent with the proposed structure. Its lubricating properties in mineral oil are recorded in Table I.
Calculated for $C_{66}H_{114}N_4C_8S_6Zn$: C, 58.0; H, 8.60; N, 4.20; S, 14.2; Zn, 4.93
Found: C, 57.5; H, 8.70; N, 2.94; S, 14.4; Zn, 5.02

Example 4

Preparation of bis[ditridecyl 2-(2-thio-1,3,4-thiadiazol-5-ylthio)-succinate-S-dibutyltin:

A solution of 3.03 grams (0.01 mole) of dibutyl tin dichloride and 12.6 grams (0.02 moles) of ditridecyl 2-(2-mercapto-1,3,4-triadiazol-5-ylthio)-succinate in 100 ml. of hexane was refluxed for six hours. The reaction was monitored by detecting hydrogen chloride liberation. When liberation of hydrogen chloride stopped, the solvent was removed by distillation. The reaction product was an amber, viscous oil. It is soluble in mineral oil and synthetic fluids. Its lubricating properties in a paraffinic oil are listed in Table I.
Calculated for $C_{72}H_{132}N_4O_8S_6Sn$: C, 57.8; H, 8.90; N, 3.75; S, 12.9; Sn, 7.95
Found: C, 58.2; H, 9.3; N, 3.77; S, 12.1; Sn, 6.38

Example 5

Preparation of bis[dioleyl 2-(2-thio-1,3,4-thiadiazol-5-ylthio)-succinate]-S-molybdenum dioxide:

A mixture of 7.66 grams (0.01 mole) of dioleyl 2-(2-mercapto-1,3,4-thiadiazol-5-ylthio)-succinate and 0.99 grams (0.005 moles) of molybdenum dichloride dioxide in 80 ml. of toluene was refluxed for six hours. After filtering and removing the solvent from the filtrate under reduced pressures, an amber, viscous

oil was obtained. The product is soluble in most non-polar solvents, mineral oils and synthetic esters. Its lubricating properties in a mineral oil and synthetic fluid are recorded in Table I.
Calculated for $C_{84}H_{144}N_4O_{10}S_6Mo$: C, 60.9; H, 8.68; N, 3.38; S, 11.6; Mo, 5.7
Found: C, 59.5; H, 9.57; N, 3.07 S, 11.4; Mo, 3.2

Example 6

Preparation of bis[ditridecyl 2-(2-thio-1,3,4-thiadiazol-5-ylthio)-succinate]-S-molybdenum dioxide:

A mixture of 7.87 grams (0.0125 moles) of ditridecyl 2-(2-mercapto-1,3,4-thiadiazol-5-ylthio)-succinate and 1.24 grams (0.00625 moles) of molybdenum dichloroide dioxide in 30 ml. of tetrahydrofuran was refluxed for three hours. The solvent was removed under reduced pressures and an amber, viscous oil was obtained. The reaction product is soluble in mineral oils and various synthetic fluid. Selected lubricating properties of this product in a mineral oil are recorded in Table I.
Calculated for $C_{64}H_{114}N_4O_8S_6Mo$: C, 55.4; H, 8.21; N, 4.0; S, 13.8
Found: C, 51.6; H, 8.37; N, 3.99 S, 13.6

Example 7

Preparation of bis[ditridecyl 2-(2-thio-1,3,4-thiadiazol-5-ylthio)-succinate]-S-cobalt(2+):

The title compound was prepared in the same manner as described in Example 3. The reaction product is a viscous oil. Its lubricating properties in a mineral oil are listed in Table I.
Calculated for $C_{64}H_{144}N_4O_8S_6Co$: C, 58.3; H, 8.70; N, 4.24; S, 14.6; C0, 4.4
Found: C, 58.4; H, 9.00; N, 4.79; S, 12.9; Co, 3.2

Example 8

Preparation of bis[ditridecyl 2-(2-thio-1,3,4-thiadiazol-5-ylthio)-succinate]-S-nickel(2+):

The title compound was prepared in the same manner as described in Example 3 by reacting ditridecyl 2-(2-mercapto-1,3,4-thiadiazol-5-ylthio)-succinate with Nickel (II) chloride in toluene. The reaction product is a viscous oil.
Calculated for $C_{64}H_{114}N_4O_8S_6Ni$: C, 58.3; H, 8.70; N, 4.24; S, 14.6
Found: C, 58.3; H, 9.04; N, 4.62; S, 12.6

Example 9

Preparation of N,N′-dioleyl-2-(2-mercapto-1,3,4-thiadiazol-5-ylthio)-succinamide:

The title compound was prepared by reacting 2-(2-mercapto-1,3,4-thiadiazol-5-ylthio)-succinic anhydride and oleylamine in 1:2 molar ratio in refluxing xylene for 14 hours. After removing the solvent, the reaction product is a brown, viscous oil and soluble in most non-polar solvents and mineral oils. Its IR spectrum and elemental analysis are consistent with the proposed structure.
Calculated for $C_{42}H_{76}N_4O_2S_3$: C, 65.9; H, 9.90; N, 7.30; S, 12.5
Found: C, 65.7; H, 10.7; N, 7.22; S, 12.2

Example 10

Preparation of bis[N,N′,-dioleyl-2-(2-thio-1,3,4-thiadiazol-5-ylthio)-succinamide]-S-zinc(2+):

The title compound was prepared by refluxing a mixture of 2-(2-mercapto-1,3,4-thiadiazol-5-ylthio)-succinic anhydride and zinc carbonate in 2:1 molar ratio in xylene for three hours. Experimental details and working up of the reaction product were similar to those described in Example 3. The reaction product is a light brown, viscous oil. Its lubricating characteristic in a mineral oil are recoreded in Table I and antioxidant properties by high pressure DSC are recorded in Table II.
Calculated for $C_{84}H_{150}N_8O_4S_6Zn$: C, 63.0; H, 9.40; N, 7.03; S, 12.10
Found: C, 63.9; H, 10.1; N, 6.75; S, 10.0

Example 11

Preparation of bis[N-(2-ethylhexyl)-2-(2-thio-1,3,4-thiadiazol-5-ylthio)-succinimide]-S-molybdenum dioxide:

A mixture of N-(2-ethylhexyl)-2-(2-mercapto-1,3,4-thiadiazol-5-ylthio)-succinimide and molybdenum dichloride dioxide in 2:1 molar ratio was refluxted in xylene for three hours. The solvent was removed by

distillation under reduced pressure and the reaction product was dark green oil. The wear preventive characteristics of the reaction product in a mineral oil are listed in Table I.
Calculated for $C_{28}H_{40}N_6O_6S_6Mo$: C, 39.6; H, 4.75; S, 22.8
Found: C, 40.6; H, 4.91; S, 22.1

Example 12

Preparation of diisocetyl 2-(2-mercapto-1,3,4-thiadiazol-5-ylthio)-succinate:

A sample of the above title compound was prepared by reacting 2-(2-mercapto-1,3,4-thiadiazol-5-ylthio)-succinic anhydride with isocetyl alcohol (1:2 molar ratio) as described in Example 2. The reaction was used for subsequent reactions with various metal salts.

Example 13

Preparation of bis[diisocetyl 2-(2-thio-1,3,4-thiadiazol-5-ylthio)-succinate]-S-zinc(2+):

A mixture of 183 grams (0.2 moles) of diisocetyl 2-(2-mercapto-1,3,4-thiadiazol-5-ylthio)-succinate and 27.4 grams (0.125 moles) of zinc acetate dihydrate in xylene was refluxed for seven hours. The reaction mixture was filtered and the filtrate was subjected to distillation to remove solvent and other byproducts. The reaction product was a viscous, brown oil and was soluble in various syntehtic fluids and mineral oils. its lubricating properties in a paraffinic mineral oil are listed in Table I and antioxidant properties are recorded in Table II.
Calculated for $C_{76}H_{138}N_4O_8S_6Zn$: C, 61.0; H, 9.24; N, 3.75; S, 12.85
Found: C, 60.5; H, 8.95; N, 3.70; S, 13.70

TABLE I
Lubricating Performance of Various Compositions

| Composition | Shell Four Ball EP Properties (ASTM D 2596) Weld Pt., kg | Load Wear Index | Wear Preventive Characteristics (ASTM D 2266) Scar Diam., mm[1] |
|---|---|---|---|
| Paraffinic Mineral Oil (P.O.)[2] | 80 | | 0.85 |
| Naphthenic Mineral Oil (N.O.)[3] | 80 | | 0.75 |
| Synthetic Ester (S.E.)[4] | 100 | | |
| 1% Zinc Complex (Example 3) in P.O. | 160–200 | 35.6 | 0.50 |
| 1% Zinc Complex (Example 3) in N.O. | 160 | | 0.48 |
| 1% Tin Complex (Example 4) in P.O. | 160 | 29.0 | 0.85 |
| 1% Mo Complex (Example 5) in P.O. | 160 | 35.0 | 0.53–0.60 |
| 1% Mo Complex (Example 5) in S.E. | 200 | | |
| 1% Zn Complex (Example 10) in P.O. | | | 0.50 |
| 1% Mo Complex (Example 12) in P.O. | | | 0.58 |
| 1% Zn Isocetyl ester (Example 14) in P.O. | 160 | 24.1 | 0.48 |
| 3% Zn Isocetyl ester (Example 14) in P.O. | 160 | 31.1 | 0.65 |

1. 75°C (167°F), 40 kg, 1200 rpm for one hour.
2. Paraffinic straight mineral oil, 155 SUS at 37.8°C (100°F).
3. Naphthenic straight mineral oil, 110 SUS at 37.8°C (100°F).
4. Pentaerythrital ester.

TABLE II

Evaluation of Antioxidant Properties of Selected Compositions in Paraffinic Mineral Oil by High Pressure DSC Under 500 PSI Oxygen at 185°C[1]

| Composition | Induction Time min. |
|---|---|
| Paraffinic Oil (Base Oil) | 1.90 |
| Base Oil + 1% Zn diamide (Example 10) | 72.9 |
| Base Oil + 1% ZDDP[2] | 109.0 |
| Base Oil + 1% Zn diester (Example 14) | 118.0 |

1. Sample was placed in a Knudsen cell.
2. Zinc diamyldithiophosphate.

Example 15

Preparation of Potassium salt of ditridecyl 2-(3-mercapto-1,2,4-thiadiazol-5-ylthio)-succinate:

A solution of 10.4g. (0.025 mole) of ditridecyl 2-chlorosuccinate in 50 ml. of absolute ethanol is added dropwise to a stirred cloudy solution of 11.3 g. (0.05 mole) of the dipotassium salt of 3,5-dimercapto-1,2,4-thiadiazole (prepared according to procedure of W.A. Thaler and J.R. McDivitt; J. Org. Chem. 36, 14-18, 1971) in 200 ml. of absolute ethanol, over a period of 10 minutes. No rise in temperature is observed. The cloudy mixture is refluxed for 18 hours.

The insoluble off white solid is filtered off, washed twice with ice cold ethanol then dried at 60° under reduced pressure to obtain 2.1g. Calculated amount of KCL-by-product; 1.86g. Almost completely soluble in water but insoluble in acetone.

The solvent of the filtrate is removed at 60° and reduced pressure and the yellow residue is dissolved in 100 ml. of distilled water, giving cloudy solution. The aquous solution is extracted with 4 x 50 ml. portions of ethyl, acetate, dried with sodium sulfate and the solvent stripped off as above to obtain 18.2 g. of a yellowish brown liquid residue, which has an offensive odor similar to that of the dipotassium salt of 3,5 -dimercapto-1,2,4-thiadiazole. The residue is treated with 125 ml.$H_2O$ & 20% HCL solution to $_pH$ 1 resulting in the separation of oil. The oil is collected and dissolved in 150 ml.ether. The ether solution is washed with 3 x 50 ml. of distilled water, dried with sodium sulfate then heated on a steam bath to remove solvent. The liquid residue after drying at 60°c under reduced pressure weigh 8.1g. Again, it has offensive odor, but much less than before.

The liquid residue is treated with 200 ml. of distilled water and 10% KOH to $_pH$ 14. The alkaline mixture is extracted with 1 x 100 ml. of either and the ether extract is in turn washed with 50 ml. of distilled water. The ether extract is dried with sodium sulfate then heated on a steam bath to remove solvent.

The liquid residue is mixed with 300 ml. of distilled water, forming a pale yellow emulsion-like solution. The solution is heated to 55° then allowed to cool to room temperature, followed by extraction with 2 x 100 ml. of ether. The ether extract is dried with sodium sulfate then heated on a steam bath to remove solvent and volatiles. The residue is dried at 80° and 5-10 mm pressure to obtain a light yellowish brown liquid product.

Infrared spectrum is consistent with the proposed structure.

Example 16

Preparation of bis[ditridecyl 2-(3-thio-1,2,4-thiodiazol-5-ylthio)-succinate]-S-Zinc (2+):

The above compound can be similarly prepared as described in Example 3 by reaction of potassium salt of ditridecyl 2-(3-mercapto-1,2,4-thiadiazol-5-ylthio)-succinate with zinc chloride in toluene.

**Claims**

1. A compound of the structure

$$\left[ Z - S - Q - S \right]_x M$$

wherein:
Q is a bivalent thiadiazole ring moiety selected from the group consisting essentially of 1,3,4-thiadiazole; 1,2,4-thiadiazole; 1,2,3-thiadiazole; and 1,2,5-thiadiazole;
Z is a succinate group of the structure

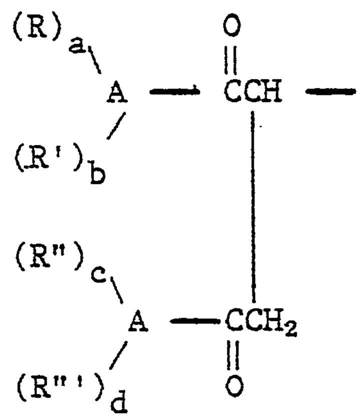

or

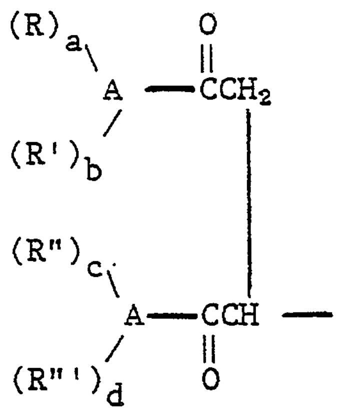

,

wherein:

A is an oxygen or nitrogen atom, with the proviso: when A is oxygen a is 1, b is zero, c is 1, and d is zero; and when A is nitrogen a, b, c, and d are each 1;
R, R′, R″, and R‴ are each independently selected from the group consisting essentially of hydrogen, alkyl of 1 through 22 carbon atoms, and branched or straight chain alkenyl of 2 through 22 carbon atoms; with the further proviso that the number one and four carbon atoms of the succinate group Z can be linked by a single A in which case when A is oxygen a, b, c, and d are zero, and when A is nitrogen a is one and b, c, and d are zero;
M is a metal ion selected from from Groups Ib to Vb, and transition elements of the Periodic Table and $MoO2^{+2}$; and x is a whole number equal to the valence of M.

2. The compound as defined in claim 1 wherein M is selected from the group consisting of zinc, copper with a valence state of 2, cobalt with a valence state of 2, $MoO_2$ with a valence state of 2, aluminum, and antimony with a valence state of 3.

3. The compound as defined in claim 1 wherein R, R′, R″, and R‴ are each independently selected from the group consisting essentially of decyl, tridecyl, oleyl, 2-ethylhexyl, and isocetyl.

4. The compound as defined in claim 1 wherein Q is the 1,3,4-thiadiazole moiety.

5. The compound as defined in claim 1 of the name bis[ditridecyl 2-(2-thio-1,3,4-thiadiazol-5-ylthio)-succinate]-S-zinc(2+).

6. The compound as defined in claim 1 of the name bis[ditridecyl 2-(2-thio-1,3,4-thiadiazol-5-ylthio)-succinate]-S-dibutyltin.

7. The compound as defined in claim 1 of the name bis[dioleyl 2-(2-thio-1,3,4-thiadiazol-5-ylthio)-succinate]-S-molybdenum dioxide.

8. The compound as defined in claim 1 of the name bis[ditridecyl 2-(2-thio-1,3,4-thiadiazol-5-ylthio)-succinate]-S-molybdenum dioxide.

9. The compound as defined in claim 1 of the name bis[ditridecyl 2-(2-thio-1,3,4-thiadiazol-5-ylthio)-succinate]-S-cobalt(2+).

10. The compound as defined in claim 1 of the name bis[ditridecyl 2(2-thio-1,3,4-thiadiazol-5-ylthio)-succinate]-S-nickel(2+).

11. The compound as defined in claim 1 of the name bis[N,N'-dioleyl-2-(2-thio-1,3,4-thiadiazol-5-ylthio)-succinamide]-S-zinc(2+).

12. The compound as defined in claim 1 of the name bis[N-(2-ethylhexyl)-2-(2-thio-1,3,4-thiadiazol-5-ylthio)-succinimide]-S-molybdenum dioxide.

13. The compound as defined in claim 1 of the name bis[diisocetyl 2-(2-thio-1,3,4-thiadiazol-5-ylthio)-succinate]-S-zinc(2+).

14. A lubricant comprising a major amount of a grease or oil of lubricating viscosity and a minor amount of a compound of claim 1 to 13 as a lubricant additive to provide enhanced properties to said grease or oil.

**Patentansprüche**

1. Verbindung der Struktur

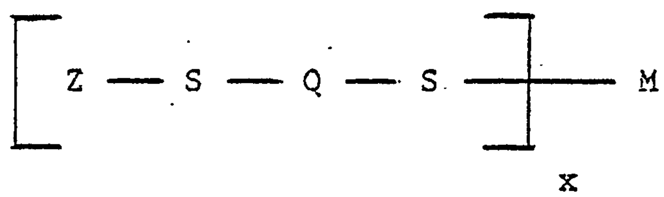

worin:
Q eine zweiwertige Thiadiazol-Ringgruppierung, ausgewählt aus der Gruppe bestehend im wesentlichen aus 1,3,4-Thiadiazol, 1,2,4-Thiadiazol, 1,2,3-Thiadiazol und 1,2,5-Thiadiazol, ist;
Z eine Succinatgruppe der Struktur

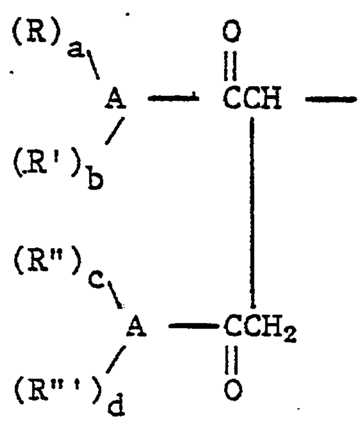

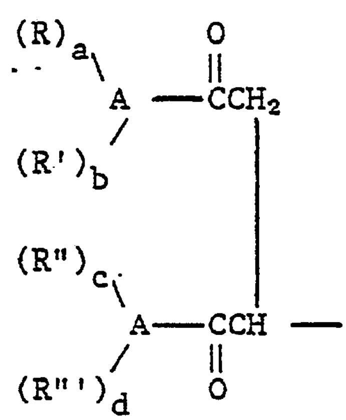

oder

ist, worin:
A für ein Sauerstoff- oder Stickstoffatom steht, mit der Maßgabe, daß, wenn A für Sauerstoff steht, a den Wert 1 hat, b den Wert Null hat, c den Wert 1 hat und d den Wert Null hat, und wenn A für Stickstoff steht, a, b, c und d jeweils den Wert 1 haben;

R, R′, R″ und R‴ unabhängig voneinander aus der Gruppe bestehend im wesentlichen aus Wasserstoff, Alkyl mit 1 bis 22 Kohlenstoffatomen und verzweigtes oder geradkettiges Alkenyl mit 2 bis 22 Kohlenstoffatomen ausgewählt ist, mit der weiteren Maßgabe, daß die Kohlenstoffatome Nummer eins und vier der Succinatgruppe Z durch ein einziges A verbunden sein können, in welchem Falle, wenn A für Sauerstoff steht, a, b, c und d den Wert Null haben, und wenn A für Stickstoff steht, a den Wert 1 hat und b, c und d jeweils den Wert Null haben;
M ein Metallion, ausgewählt aus den Gruppen Ib bis Vb und Übergangselementen des Periodischen Systems und $MoO2^{+2}$, ist und x eine ganze Zahl ist, die der Wertigkeit von M gleich ist.

2. Verbindung nach Anspruch 1, dadurch gekennzeichnet, daß M aus der Gruppe bestehend aus Zink, Kupfer mit dem Wertigkeitszustand 2, Kobalt mit dem Wertigkeitszustand 2, $MoO_2$ mit einem Wertigkeitszustand von 2, Aluminium und Antimon mit einem Wertigkeitszustand von 3 ausgewählt ist.

3. Verbindung nach Anspruch 1, dadurch gekennzeichnet, daß R, R′, R″ und R‴ jeweils unabhängig aus der Gruppe bestehend im wesentlichen aus Decyl, Tridecyl, Oleyl, 2-Ethylhexyl und Isocetyl ausgewählt sind.

4. Verbindung nach Anspruch 1, dadurch gekennzeichnet, daß Q die 1,3,4-Thiadiazol-Gruppierung ist.

5. Verbindung nach Anspruch 1 mit dem Namen Bis-[ditridecyl-2-(2-thio-1,3,4-thiadiazol-5-ylthio)-succinat]-S-zink-(2+).

6. Verbindung nach Anspruch 1 mit dem Namen Bis-[ditridecyl-2-(2-thio-1,3,4-thiadiazol-5-ylthio)-succinat]-S-dibutylzinn.

7. Verbindung nach Anspruch 1 mit dem Namen Bis-[dioleyl-2-(2-thio-1,3,4-thiadiazol-5-ylthio)-succinat]-S-molybdändioxid.

8. Verbindung nach Anspruch 1 mit dem Namen Bis-[ditridecyl-2-(2-thio-1,3-4-thiadiazol-5-ylthio)-succinat]-S-molybdändioxid.

9. Verbindung nach Anspruch 1 mit dem Namen Bis-[ditridecyl-2-(2-thio-1,3,4-thiadiazol-5-ylthio)-succinat]-S-kobalt(2+).

10. Verbindung nach Anspruch 1 mit dem Namen Bis-[ditridecyl-2-(2-thio-1,3,4-thiadiazol-5-ylthio)-succinat]-S-nickel(2+).

11. Verbindung nach Anspruch 1 mit dem Namen Bis-[N,N′-dioleyl-2-(2-thio-1,3,4-thiadiazol-5-ylthio)-succinamid]-S-zink(2+).

12. Verbindung nach Anspruch 1 mit dem Namen Bis-[N-(2-ethylhexyl)-3-(2-thio-1,3,4-thiadiazol-5-ylthio)-succinimid]-S-molybdän-dioxid.

13. Verbindung nach Anspruch 1 mit dem Namen Bis-[diisocetyl-2-(2-thio-1,3,4-thiadiazol-5-ylthio)-succinat]-S-zink(2+).

14. Schmiermittel, dadurch gekennzeichnet, daß es eine Hauptmenge eines Fettes oder Öls mit schmierender Viskosität und eine geringere Menge einer Verbindung nach den Ansprüchen 1 bis 13 als Schmiermitteladditiv zur Erzielung erhöhter Eigenschaften des genannten Fettes oder Öls enthält.

**Revendications**

1. Composé de formule

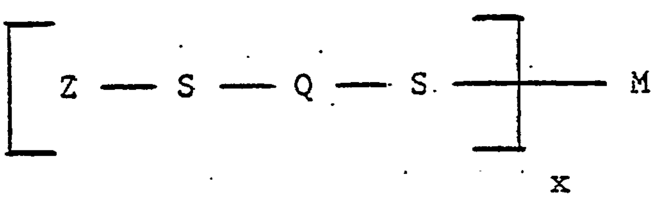

dans laquelle:
Q est un groupement bivalent à noyau de thiadiazole essentiellement choisi dans le groupe formé du 1,3,4-thiadiazole; du 1,2,4-thiadiazole; du 1,2,3-thiadiazole; et du 1,2,5-thiadiazol;
Z est un groupe succinate de formule

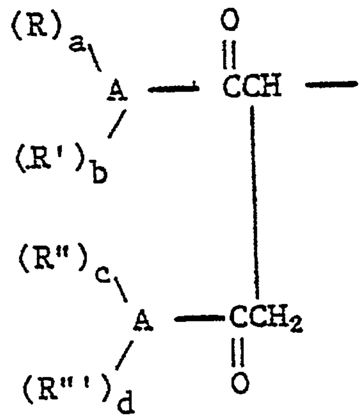

ou

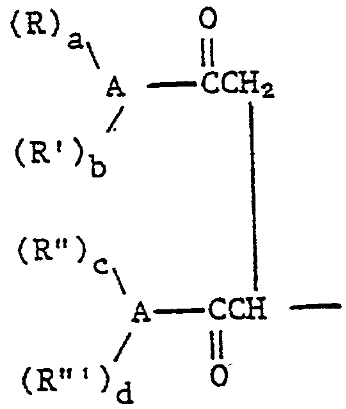

dans laquelle

A est un atome d'oxygène ou d'azote, à condition que: lorsque A représente l'oxygène, a soit égal à 1, b soit égal à zéro, c soit égal à 1 et d soit égal à zéro; et que lorsque A représente l'azote, a, b, c, et d soient tous égaux à 1;

R, R′, R″ et R‴ sont choisis chacun, indépendamment, dans le groupe essentiellement formé de l'hydrogène, de radicaux alkyle ayant 1 à 22 atomes de carbone et de radicaux alcényle à chaîne droite ou à chaîne ramifiée ayant 2 à 22 atomes de carbone; sous réserve en outre que les atomes de carbone No. 1 et No. 4 du groupe succinate Z puissent être liés par un seul atome A, auquel cas lorsque A est l'oxygène, a, b, c et d sont égaux à zéro, et lorsque A est l'azote, a a la valeur un et b, c et d sont égaux à zéro;

M est un ion métallique choisi dans les Groupes Ib à Vb et parmi les éléments de transition du Tableau Périodique et l'ion $MoO2^{+2}$; et x est un nombre entier égal à la valence de M.

2. Composé suivant la revendication 1, dans lequel M est choisi dans le groupe comprenant le zinc, le cuivre à la valence 2, le cobalt à la valence 2, $MoO_2$ à la valence 2, l'aluminium et l'antimoine à la valence 3.

3. Composé suivant la revendication 1, dans lequel R, R′, R″ et R‴ sont choisis chacun, indépendamment, dans le groupe essentiellement constitué des radicaux décyle, tridécyle, oléyle, 2-éthylhexyle et isocétyle.

4. Composé suivant la revendication 1, dans lequel Q est le groupement 1,3,4-thiadiazole.

5. Composé suivant la revendication 1, répondant au nom de bis[2-(2-thio-1,3,4-thiadiazole-5-ylthio)succinate de ditridécyle]-S-zinc(2+).

6. Composé suivant la revendication 1, répondant au nom de bis[2-(2-thio-1,3,4-thiadiazole-5-ylthio)succinate de ditridécyle]-S-dibutylétain.

7. Composé suivant la revendication 1, répondant au nom de dioxyde de bis[2-(2-thio-1,3,4-thiadiazole-5-ylthio)succinate de dioléyle]-S-molybdène.

8. Composé suivant la revendication 1, répondant au nom de dioxyde de bis[2-(2-thio-1,3,4-thiadiazole-5-ylthio)succinate de ditridécyle]-S-molybdène.

9. Composé suivant la revendication 1, répondant au nom de bis[2-(2-thio-1,3,4-thiadiazole-5-ylthio)succinate de ditridécyle]-S-cobalt(2+).

10. Composé suivant la revendication 1, répondant au nom de bis[2-(2-thio-1,3,4-thiadiazole-5-ylthio)succinate de ditridécyle]-S-nickel(2+).

11. Composé suivant la revendication 1, répondant au nom de bis[N,N'-dioléyl-2-(2-thio-1,3,4-thiadiazole-5-ylthio)succinamide]-S-zinc(2+).

12. Composé suivant la revendication 1, répondant au nom de dioxyde de bis[N-(2-éthylhexyl)-3-(2-thio-1,3,4-thiadiazole-5-ylthio)succinimide]-S-molybdène.

13. Composé suivant la revendication 1, répondant au nom de bis[2-(2-thio-1,3,4-thiadiazole-5-ylthio)succinate de diisocétyle]-S-zinc(2+).

14. Lubrifiant comprenant une quantité dominante d'une graisse ou d'une huile de viscosité propre à la lubrification et une quantité secondaire d'un composé suivant les revendications 1 à 13 comme additif pour lubrifiant destiné à améliorer les propriétés de cette graisse ou de cette huile.